(19) Europäisches Patentamt
European Patent Office
Office européen des brevets

(11) Veröffentlichungsnummer: **0 077 946**
**B1**

# (12) EUROPÄISCHE PATENTSCHRIFT

(45) Veröffentlichungstag der Patentschrift: **29.01.86**

(51) Int. Cl.⁴: **C 01 B 33/20**, C 01 B 35/10, B 01 J 29/04

(21) Anmeldenummer: **82109286.3**

(22) Anmeldetag: **07.10.82**

(54) **Borosilikatzeolithe - ZBH und Verfahren zur Herstellung kristalliner Borosilikatzeolithe (ZBH) und Verwendung als Katalysator.**

(30) Priorität: **15.10.81 DE 3140895**

(43) Veröffentlichungstag der Anmeldung: **04.05.83 Patentblatt 83/18**

(45) Bekanntmachung des Hinweises auf die Patenterteilung: **29.01.86 Patentblatt 86/5**

(84) Benannte Vertragsstaaten: **BE DE FR GB IT NL**

(56) Entgegenhaltungen:
**EP-A-0 051 741**
**DE-A-2 746 790**

(73) Patentinhaber: **BASF Aktiengesellschaft, Carl-Bosch-Strasse 38, D-6700 Ludwigshafen (DE)**

(72) Erfinder: **Hoelderich, Wolfgang, Dr., Mannheimer Strasse 18 C, D-6710 Frankenthal (DE)**
Erfinder: **Mross, Wolf Dieter, Dr., Anselm-Feuerbach-Strasse 21, D-6710 Frankenthal (DE)**
Erfinder: **Schwarzmann, Matthias, Dr., Carl-Bosch-Strasse 54, D-6703 Limburgerhof (DE)**

EP 0 077 946 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Borosilikatzeolithe – ZBH und ein Verfahren zur Herstellung kristalliner Borosilikatzeolithe – ZBH vom Pentasiltyp in reinem oder wässrigem etherischen Medium ohne Verwendung von Aminen sowie die Verwendung der Katalysatoren für die Umwandlung von Methanol oder Dimethylether zu niederen Olefinen.

Bor-Zeolithe sind bisher in der Natur nicht gefunden worden. Man erhält sie nur auf synthetischem Wege (DE-Offenlegungsschrift 27 46 790). Al-haltigel zeolithische Materialien, sowohl natürlicher als auch synthetischer Herkunft, haben sich als katalytisch wirksam erwiesen für verschiedene Arten von Kohlenwasserstoffumwandlungen, z.B. in der Technik zum Cracken von Kohlenwasserstoffen und finden auch als Ionenaustauscher und Molekularsiebe technische Verwendung.

Allgemein besitzen Zeolithe eine hochgeordnete Struktur mit einem starren dreidimensionalen Netzwerk von $SiO_4$- und $MeO_4$-Tetraedern, wobei Me z.B. Al, Fe, Ga und B sein kann. Diese Tetraeder sind durch gemeinsame Sauerstoffatome verbunden. Das Verhältnis der Si- und Me-Atome zu Sauerstoff beträgt 1:2.
Die Elektrovalenz der dreiwertige Elemente Me enthaltenden Tetraeder ist durch Einschluss von Kationen in den Kristall z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekeln besetzt.

In den letzten Jahren gewannen kristalline Aluminosilikate mit einem hohen $SiO_2/Al_2O_3$-Verhältnis $\geq 11$ zunehmendes Interesse. Derartige Zeolithe besitzen Pentasilstruktur und zeichnen sich durch hohe thermische Stabilität und ausserordentlich hohe Acidität aus. Die Synthese dieser Zeolithe erfolgt in bekannter Weise aus einer Silicium- und einer Aluminiumkomponente in Gegenwart von voluminösen quaternären organischen Ammonium-bzw. Phosphoniumverbindungen und Alkaliverbindungen als Mineralisierungsmittel. Ein derartiges Verfahren ist in US-Patentschrift 3 702 886 beschrieben. Die grossen Ammonium- bzw. Phosphoniumionen sollen als Templat für die gewünschte Zeolithstruktur wirken und ermöglichen die Synthese von Aluminium-Zeolithen mit $SiO_2/Al_2O_3$-Verhältnissen z.B. von 100 bis zu 3000.

Bor-Zeolithe mit Pentasil-Struktur wurden bisher nur in Gegenwart von Aminen hergestellt (DE-Offenlegungsschrift 27 46 790). Die Verwendung der quaternären Amine ist technisch problematisch.
Gegenstand der Erfindung sind nun neuartige Borosilikatzeolithe – ZBH der allgemeinen Formel

$$M_{2/n}O : B_2O_3 : x\,SiO_2 : y\,H_2O,$$

worin M zumindest ein Kation mit einer Wertigkeit n bedeutet, $x \geq 10$ beträgt und y zwischen 0 und 80 liegt, die das folgende Röntgenbeugungsdiagramm mit den wichtigsten Beugungslinien aufweisen:

| Interplanarer Abstand d (Å) | Relative Intensität I/I° |
|---|---|
| 11.0746 ± 0,15 | 100 |
| 9.9725 | 68 |
| 9.7271 | 19 |
| 3.8322 | 96 |
| 3.7980 | 70 |
| 3.7282 | 30 |
| 3.6940 | 46 |
| 3.6254 | 25 |
| 1.9999 ± 0,07 | 10 |
| 1.9825 | 9 |

Es wurde gefunden, dass man diese Borosilikatzeolithe – ZBH vom Pentasiltyp durch hydrothermale Kristallisation von Siliciumdioxid und Borsäure in Gegenwart von Alkalisalzen erhält, wenn man die Kristallisation bei Temperaturen von 80 bis 140°C in etherischer oder wässrig-etherischer Mischung ausführt.

Das Molverhältnis von $SiO_2$ zu $B_2O_3$ in der Reaktionsmischung wird dabei zwischen 3 und 300, vorzugsweise bei 10 bis 50 eingestellt.

Eine bevorzugte Ausführungsform zur Herstellung dieser aminfreien Borosilikatzeolithe – ZBH besteht darin, dass man eine Reaktionsmischung aus $SiO_2$, z.B. eine käufliche pyrogene Kieselsäure, $H_3BO_3$ und NaOH in einer Ether/ Wasser-Mischung von 50 : 50 Gew.-% 5 bis 7 Tage bei 110 bis 130°C unter autogenem Druck in einem Rührautoklaven umsetzt. Das zunächst erhaltene Reaktionsprodukt kann vor der Calcinierung noch Ether enthalten anstelle der in den intrakristallinen Poren enthaltenden $H_2O$-Molekeln. Alle Reste organischer Verbindungen werden durch Trocknen und Calcinieren entfernt, so dass nur noch die freien Alkaliionen im Zeolith enthalten sind.

Der Anteil an Alkalihydroxid liegt bei 1 bis 3 Mol pro Mol Äquivalent $B_2O_3$, bevorzugt bei 1,7 Mol.

Es ist zweckmässig, Impfkristalle zur Reaktionsführung und -beschleunigung zuzusetzen.

Als Lösungsmittel kann man allgemein Ether, sowohl lineare als auch cyclische Ether mit einer $(-CH_2-CH_2-O-)$-Gruppierung wie Mono-, Di-, Tri- und Tetraethylenglykoldimethylether, Diethylether, Tetrahydrofuran, Dioxan oder ein Gemisch von Tri- bis Dekaethylenglykol-methylisopropylether des mittleren Ethoxylierungsgrades 5,5 der Formel $CH_3O-(CH_2-CH_2-O)_{5,5}-C_3H_7$, Diethylenglykolmethyl-isopropylether oder ein Gemisch von Polyethylenglykol-methyl-isopropylethern des mittleren Ethoxylierungsgrades 5,5 der Formel $CH_3O(-CH_2-CH_2-O)_{5,5}$ oder ein Gemisch von ethoxylierten Oxoalkoholen mit mittlerem Molekulargewicht von 200, 600, 6000 sowie lineare Ether mit einer $(CH_3-CH-CH_2-O)$-Gruppierung, sowie lineare Ether mit einer $(-CH_2-O)$-Gruppierung wie Dimethoxymethan. Besonders mit den Glymen erhält man gut geformte Kristalle der ZBH-Bor-Zeolithe von der Grösse von 1-5$\mu$. Dies ist ebenfalls ein Vorteil dieser Herstellungsweise in Ethern.

Die molare Zusammensetzung der Reaktionsmischung wird wie folgt eingestellt:

$SiO_2/B_2O_3$ = 3–300, vorzugsweise 10–50
$M_2O/B_2O_3$ = >1, vorzugsweise 1,3–2
$ROR/B_2O_3$ = 18–390,

wobei M=Alkali, insbesondere Na, und ROR einen Ether bedeutet. Die Konzentration der Ether in $H_2O$ kann zwischen 10 und 100 Gew.-% liegen, bevorzugt 50 Gew.-%

Das erfindungsgemässe Verfahren wird bei Reaktionstemperaturen im Bereich von 80-140°C, vorzugsweise zwischen 110 und 130°C, und vorteilhafterweise unter dem Eigendruck in einem Rührautoklaven aus Edelstahl während 3 bis 7 Tagen durchgeführt.

Nach der Reaktion wird das Reaktionsprodukt zweckmässigerweise abfiltriert, mit Wasser gut ausgewaschen und bei 110°C/16 h getrocknet. Anschliessend wird das Produkt bei 550°C/20 h calciniert, um den noch eingeschlossenen Ether herauszubrennen und den Zeolith zu dehydratisieren. Die Überführung der Alkali-Form des Zeolithen in die katalytisch aktive H-Form kann nach bekannten Austauschverfahren z.B. mit Ammoniumsalzen erfolgen. Die abgetrennte Mutterlauge kann man für weitere Synthesen verwenden.

Die erfindungsgemäss in Ether hergestellten Borosilikatzeolithe – ZBH zeigen folgende Röntgenbeugungslinien:

Tabelle 1

| Interplanarer Abstand d (Å) | Relative Intensität I/I° | Interplanarer Abstand d (Å) | Relative Intensität I/I° |
|---|---|---|---|
| 11.0746 ± 0,15 | 100 | 2.9259 | 7 |
| 9.9725 | 68 | 2.8543 | 4 |
| 9.7271 | 19 | 2.7151 | 5 |
| 6.6567 | 10 | 2.5928 | 5 |
| 6.3220 | 17 | 2.4984 | 3 |
| 5.9489 ± 0,1 | 22 | 2.4746 | 5 |
| 5.6740 | 11 | 2.4013 | 4 |
| 5.5405 | 18 | 2.3831 | 5 |
| 5.0022 | 10 | 2.3106 | 3 |
| 4.9479 | 10 | 1.9999 | 10 |
| 4.5883 | 8 | 1.9825 | 9 |
| 4.3352 | 10 | 1.9418 | 3 |
| 4.2392 | 11 | 1.9062 | 3 |
| 3.9747 ± 0,07 | 5 | 1.8621 | 5 |
| 3.8322 | 96 | 1.8289 | 3 |
| 3.7980 | 70 | 1.7567 | 3 |
| 3.7282 | 30 | 1.6615 | 3 |
| 3.6940 | 46 | 1.6544 | 3 |
| 3.6254 | 25 | 1.6501 | 3 |
| 3.4652 | 5 | 1.4807 | 3 |
| 3.4174 | 9 | 1.4539 | 4 |
| 3.3654 | 6 | 1.4377 | 4 |
| 3.3371 | 10 | 1.4149 | 4 |
| 3.2907 | 10 | 1.4046 | 3 |
| 3.2429 | 3 | 1.3864 | 4 |
| 3.1188 | 4 | 1.3738 | 4 |
| 3.0303 | 12 | 1.3556 | 3 |
| 2.9691 | 13 | | |

Das Röntgenbeugungsdiagramm wird mit einem automatischen Pulverdiffraktometer APD-10 hergestellt. Es wird Kupferstrahlung zusammen mit einem Graphitmonochromator verwendet.

Das für die in Ether hergestellten Borosilikatzeolithe - ZBH typische Röntgenbeugungsmuster weist diese als Mitglieder der Pentasilfamilie aus.

In den erfindungsgemässen Borosilikatzeolithen - ZBH ist das Bor in die tetraedrischen Gitterplätze des Kristallgerüstes eingebaut. Der Einbau von Bor in das Kristallgerüst kommt in der Verschiebung der Röntgenbeugungslinien nach kleineren d-Werten im Vergleich zu entsprechenden Aluminosilikatzeolithen zum Ausdruck (Tabelle 2); die Bindungslänge einer B-O-Bindung ist erheblich kürzer als der Al-O-Abstand. Der Einbau des Bors in das Kristallgerüst bewirkt daher eine Kontraktion der Elementarzelle, d.h. eine Verschiebung der d-Abstände nach kleineren Werten im Vergleich zu entsprechenden Aluminiumsilikatzeolithen bzw. im Vergleich zu den d-Abständen des Silicalith.

Ein Vergleich der Netzebenenabstände der hier beschriebenen Borzeolithe mit denen des ZSM-5 zeigt, dass die d-Werte (Å) beim Bor-Zeolith kleiner sind als beim Aluminosilikat ZSM-5 (Tabelle 2).

Tabelle 2

| ZBH | | ZBH | |
|---|---|---|---|
| D | I/I | D | I/I |
| 11.0746 | 100 | 2.9259 | 7 |
| 9.9725 | 68 | 2.8543 | 4 |
| 9.7271 | 19 | 2.7151 | 5 |
| 6.6567 | 10 | 2.5928 | 5 |
| 6.3220 | 17 | 2.4984 | 3 |
| 5.9489 | 22 | 2.4746 | 5 |
| 5.6740 | 11 | 2.4013 | 4 |
| 5.5405 | 18 | 2.3831 | 5 |
| 5.0022 | 10 | 2.3106 | 3 |
| 4.9479 | 10 | 1.9999 | 10 |
| 4.5883 | 8 | 1.9825 | 9 |
| 4.3352 | 10 | 1.9418 | 3 |
| 4.2392 | 11 | 1.9062 | 3 |
| 3.9747 | 5 | 1.8621 | 5 |
| 3.8322 | 96 | 1.8289 | 3 |
| 3.7980 | 70 | 1.7567 | 3 |
| 3.7282 | 30 | 1.6615 | 3 |
| 3.6940 | 46 | 1.6544 | 3 |
| 3.6254 | 25 | 1.6501 | 3 |
| 3.4652 | 5 | 1.4807 | 3 |
| 3.4174 | 9 | 1.4539 | 4 |
| 3.3654 | 6 | 1.4377 | 4 |
| 3.3371 | 10 | 1.4149 | 4 |
| 3.2907 | 10 | 1.4046 | 3 |
| 3.2429 | 3 | 1.3864 | 4 |
| 3.1188 | 4 | 1.3738 | 4 |
| 3.0303 | 12 | 1.3556 | 3 |
| 2.9691 | 13 | | |

Tabelle 2 (Fortsetzung)

| ZSM-5[1] d(A) | I | AMS-1 B[2] d(Å) | Relative Intensität |
|---|---|---|---|
| 11,36 | s | 11,3 ± 0,2 | 38 |
| 10,20 | ms | 10,1 ± 0,2 | 30 |
| 9,90 | - | 6,01 ± 0,07 | 14 |
| 9,14 | vw | 4,35 ± 0,05 | 11 |
| 7,54 | w | 4,26 ± 0,05 | 14 |
| 7,17 | w | 3,84 ± 0,05 | 100 |
| 6,79 | vw | 3,72 ± 0,05 | 52 |
| 6,06 | w | 3,65 ± 0,05 | 31 |
| 5,77 | w | 3,44 ± 0,05 | 14 |
| 5,63 | w | 3,33 ± 0,05 | 16 |
| 5,42 | vw | 3,04 ± 0,05 | 16 |
| 5,19 | vw | 2,97 ± 0,02 | 22 |
| 5,05 | w | 2,48 ± 0,02 | 11 |
| 4,65 | w | 1,99 ± 0,02 | 20 |
| 4,40 | w | 1,66 ± 0,02 | 12 |
| 4,30 | w | | |
| 4,12 | vw | | |
| 4,04 | vw | | |
| 3,84 | vs | | |
| 3,74 | vs | | |
| 3,62 | s | | |
| 3,50 | w | | |
| 3,46 | w | | |
| 3,33 | w | | |
| 3,27 | vw | | |
| 3,07 | w | | |
| 3,00 | m | | |

[1] Zeolite, Molecular Sieves, Donald W. Breck, Wiley-Interscience-Verlag, S. 373 (1974)
[2] DE-OS 27 46 790, Standard Oil Co.

Die hier beschriebenen kristallinen Borosilikatzeolithe – ZBH unterscheiden sich in ihren interplanaren Abständen (d-Werte in Å) deutlich von den Werten des Borozeolithen AMS – 1 B der Standard Oil Co. (Tabelle 2).

Auch ein Vergleich der Intensitäten der Röntgenbeugungslinien zeigt deutliche Unterschiede auf.

Die Unterschiede sowohl in den d-Werten als auch in den Intesitäten weisen die ZBH-Borosilikate als neuartige Zeolithe aus.

Die in Ethern hergestellten Borosilikatzeolithe – ZBH gehören zwar im Strukturtyp zur Pentasilfamilie, zeigen jedoch gegenüber den anderen Mitgliedern dieses Strukturtyps deutlich verschiedene Röntgenbeugungslinien und damit andere Gitterabstände.

Die nach Austausch in H-Form erhaltenen Zeolithe können mit Matrixmaterial verstrangt und als Katalysator für Kohlenwasserstoffumwandlungen wie Isomerisierungen, Cracken und Umwandlung von Alkoholen und Ethern eingesetzt werden.

Die in den Beispielen angegebenen Analysenwerte beziehen sich auf Trockenbasis. Die Substanzen werden vor der chemischen Analyse bei 550°C/20 h calciniert. Die Differenz zu 100% ergibt sich durch adsorbiertes Wasser.

## Beispiele 1–6

In 1000 g Ether/$H_2O$-Gemisch (50:50) werden bei etwa 40°C 80 g Aerosil eingerührt. 15,2 g $H_3BO_3$ werden in 200 g Ether/$H_2O$-Gemisch gelöst. Beide Lösungen werden zusammengegeben. Danach werden 14,7 g NaOH und eventuell Impfkristalle zugesetzt. Die homogen gerührte Mischung wird in einem Rührautoklaven 5 Tage bei Temperaturen zwischen 80° und 140°C unter autogenem Druck umgesetzt. Nach Abkühlen des Reaktionsgemisches wird das kristalline Produkt abfiltriert, mit 10 l Wasser ausgewaschen, 16 h bei 110°C getrocknet und 20 h bei 500°C calciniert.

Die Ergebnisse der Beispiele 1 bis 6 mit verschiedenen Lösungsmitteln sind in Tabelle 3 zusammengefasst. Die synthetisierten Bor-Zeolithe wurden ohne Verunreinigungen erhalten.

In Tabelle 4 sind die Reihenfolgen der Beispiele und die Röntgenbeugungsdiagramme der Zeolithe angegeben, wie sie mit den verschiedenen Lösungsmitteln erhalten worden sind.

## Beispiele 7–8

Die Beispiele 7 bis 8 (Tabelle 5) zeigen die Ergebnisse bei der Herstellung von kristallinen Borosilikatzeolithen in Diethylenglykoldimethylether mit variablem $SiO_2/B_2O_3$-Verhältnis.

Tabelle 3

| Beispiel | Ether | eingesetztes $SiO_2/B_2O_3$-Verhältnis | gefunden $SiO_2$ % | gefunden $B_2O_3$ % | gefunden $SiO_2/B_2O_3$ | gefunden Na % |
|---|---|---|---|---|---|---|
| 1 | Ethylenglykoldimethylether | 11 | 86,6 | 4,58 | 22,1 | 4,3 |
| 2 | Diethylenglykoldimethylether | 11 | 87,6 | 3,5 | 29,2 | 3,1 |
| 3 | Triethylenglykoldimethylether | 11 | 79,7 | 8,28 | 11,3 | 7,2 |
| 4 | Tetraethylenglykoldimethylether | 11 | 89,3 | 3,6 | 29,0 | 4,0 |
| 5 | $CH_3O\text{-}(CH_2\text{-}CH_2\text{-}O)_{5,5}\text{-}C_3H_7$ | 11 | 92,8 | 1,17 | 92,6 | 3,05 |
| 6 | $CH_3O\text{-}(CH_2\text{-}CH_2\text{-}O)_2\text{-}C_3H_7$ | 11 | 85,0 | 6,18 | 16,0 | 3,86 |

Tabelle 4

| Beispiel 1 | | Beispiel2 | | Beispiel3 | | Beispiel 4 | | Beispiel 5 | | Beispiel 6 | |
|---|---|---|---|---|---|---|---|---|---|---|---|
| d(Å) | I/I° | d(Å) | I/I° | d(Å) | I/I° | d(Å) | I/I° | d(Å) | I/I° | d(Å) | I/I° |
| 11.0239 | 92 | 11.0746 | 100 | 10.9615 | 65 | 10.8451 | 11 | 11.0877 | 82 | 10.9802 | 88 |
| 9.9244 | 65 | 9.9725 | 68 | 9.8165 | 52 | 9.9248 | 63 | 9.9659 | 66 | 9.8640 | 63 |
| 9.6520 | 22 | 9.7271 | 19 | 6.2778 | 17 | 9.6640 | 20 | 9.7499 | 20 | 6.6145 | 8 |
| 6.2986 | 15 | 6.6567 | 10 | 5.9070 | 18 | 6.6645 | 8 | 6.3307 | 15 | 6.2757 | 15 |
| 5.9453 | 17 | 6.3220 | 17 | 4.9255 | 14 | 6.3294 | 13 | 5.9459 | 19 | 5.9344 | 21 |
| 5.6705 | 12 | 5.9489 | 22 | 4.5591 | 15 | 5.9518 | 19 | 5.6699 | 10 | 5.6334 | 11 |
| 5.5216 | 15 | 5.6740 | 11 | 3.8114 | 100 | 5.6628 | 10 | 5.5449 | 14 | 5.5386 | 14 |
| 4.9889 | 9 | 5.5405 | 18 | 3.7773 | 74 | 5.5281 | 17 | 4.9861 | 10 | 5.0769 | 4 |
| 4.9403 | 8 | 5.0022 | 10 | 3.6831 | 54 | 4.9904 | 11 | 4.6151 | 9 | 4.9609 | 10 |
| 4.5789 | 10 | 4.9479 | 10 | 3.6514 | 22 | 4.9372 | 8 | 4.5917 | 11 | 4.9298 | 9 |
| 4.4317 | 6 | 4.5883 | 8 | 3.4392 | 34 | 4.5813 | 8 | 4.3479 | 17 | 4.5711 | 7 |
| 4.3356 | 12 | 4.3352 | 10 | 3.4083 | 38 | 4.2354 | 12 | 3.8365 | 100 | 4.3218 | 10 |
| 4.2248 | 13 | 4.2392 | 11 | 3.3848 | 32 | 3.9794 | 6 | 3.8026 | 63 | 4.2193 | 13 |
| 3.9758 | 6 | 3.9747 | 5 | 3.3482 | 37 | 3.8282 | 100 | 3.7027 | 46 | 3.9654 | 6 |
| 3.8218 | 100 | 3.8322 | 96 | 3.3178 | 34 | 3.7949 | 73 | 3.6328 | 25 | 3.8215 | 100 |
| 3.7928 | 71 | 3.7980 | 70 | 3.2976 | 27 | 3.6941 | 51 | 3.4261 | 20 | 3.7915 | 70 |
| 3.6911 | 50 | 3.7282 | 30 | 3.2803 | 24 | 3.6275 | 27 | 3.4055 | 20 | 3.6864 | 47 |
| 3.6255 | 29 | 3.6940 | 46 | 3.2514 | 20 | 3.4508 | 7 | 3.3895 | 19 | 3.6154 | 29 |
| 3.4524 | 9 | 3.6254 | 25 | 3.0179 | 18 | 3.4165 | 9 | 3.3754 | 19 | 3.4486 | 7 |
| 3.4173 | 12 | 3.4652 | 5 | 2.9612 | 18 | 3.3287 | 10 | 3.3538 | 30 | 3.4141 | 8 |
| 3.3517 | 12 | 3.4174 | 9 | 2.9443 | 16 | 3.2877 | 10 | 3.3330 | 25 | 3.3737 | 6 |
| 3.3237 | 14 | 3.3654 | 6 | 2.0532 | 8 | 3.0288 | 11 | 3.3207 | 21 | 3.3563 | 6 |
| 3.2935 | 13 | 3.3371 | 10 | 1.9936 | 12 | 2.9706 | 12 | 3.2989 | 20 | 3.3223 | 10 |
| 3.0295 | 12 | 3.2907 | 10 | 1.9718 | 10 | 2.9519 | 11 | 3.2763 | 14 | 3.2823 | 11 |
| 2.9667 | 14 | 3.2429 | 3 | 1.8542 | 8 | 2.9199 | 10 | 3.2483 | 11 | 3.0269 | 12 |
| 2.9206 | 11 | 3.1188 | 4 | 1.8369 | 9 | 2.8500 | 4 | 3.0278 | 9 | 2.9642 | 14 |
| 2.7145 | 6 | 3.0303 | 12 | 1.4508 | 5 | 2.5901 | 5 | 2.9764 | 14 | 2.9233 | 8 |
| 2.5969 | 5 | 2.9691 | 13 | | | 2.5490 | 3 | 2.9662 | 11 | 2.8453 | 3 |
| 2.4732 | 6 | 2.9259 | 7 | | | 2.4718 | 5 | 2.9224 | 9 | 2.7149 | 5 |
| 2.4015 | 5 | 2.8543 | 4 | | | 2.4023 | 4 | 2.7229 | 6 | 2.5863 | 5 |
| 2.3828 | 5 | 2.7151 | 5 | | | 2.3796 | 5 | 2.6015 | 6 | 2.4977 | 3 |
| 2.0536 | 3 | 2.5928 | 5 | | | 1.9984 | 11 | 2.4077 | 5 | 2.4717 | 5 |
| 1.9980 | 11 | 2.4984 | 3 | | | 1.9782 | 10 | 2.3915 | 5 | 2.4010 | 5 |
| 1.9766 | 11 | 2.4746 | 5 | | | 1.9401 | 3 | 2.3807 | 5 | 2.3777 | 5 |
| 1.9396 | 3 | 2.4013 | 4 | | | 1.9029 | 3 | 2.0022 | 10 | 2.3043 | 2 |
| 1.9059 | 3 | 2.3831 | 5 | | | 1.8613 | 4 | 1.9887 | 7 | 1.9984 | 11 |
| 1.8597 | 4 | 2.3106 | 3 | | | 1.8175 | 2 | 1.9834 | 8 | 1.9806 | 12 |
| 1.8421 | 3 | 1.9999 | 10 | | | 1.7582 | 2 | 1.8439 | 4 | 1.9432 | 3 |
| 1.8354 | 2 | 1.9825 | 9 | | | 1.7528 | 2 | 1.8397 | 4 | 1.9074 | 3 |
| 1.8179 | 2 | 1.9418 | 3 | | | 1.7446 | 3 | 1.8324 | 4 | 1.9016 | 3 |
| 1.7589 | 3 | 1.9062 | 3 | | | 1.6610 | 3 | 1.6628 | 4 | 1.8595 | 3 |
| 1.7464 | 3 | 1.8621 | 5 | | | 1.6507 | 3 | 1.6547 | 3 | 1.7613 | 2 |
| 1.6578 | 4 | 1.8289 | 3 | | | 1.6165 | 2 | 1.4565 | 4 | 1.7575 | 3 |
| 1.6504 | 4 | 1.7567 | 3 | | | 1.6010 | 3 | 1.4544 | 0 | 1.7538 | 0 |
| 1.6029 | 3 | 1.6615 | 3 | | | 1.5519 | 2 | 1.3903 | 4 | 1.7444 | 2 |
| 1.4896 | 2 | 1.6544 | 3 | | | 1.4531 | 4 | | | 1.6605 | 4 |
| 1.4530 | 5 | 1.6501 | 3 | | | 1.4432 | 2 | | | 1.6522 | 3 |
| 1.4359 | 3 | 1.4807 | 3 | | | 1.4361 | 3 | | | 1.6168 | 2 |
| 1.4151 | 2 | 1.4539 | 4 | | | 1.4130 | 2 | | | 1.6040 | 2 |
| 1.3854 | 0 | 1.4377 | 4 | | | 1.3852 | 0 | | | 1.5557 | 2 |
| 1.3521 | 2 | 1.4149 | 4 | | | 1.3547 | 2 | | | 1.5108 | 2 |
| | | 1.4046 | 3 | | | 1.3524 | 2 | | | 1.4923 | 2 |
| | | 1.3864 | 4 | | | | | | | 1.4541 | 4 |
| | | 1.3738 | 4 | | | | | | | 1.4378 | 3 |
| | | 1.3556 | 3 | | | | | | | 1.4031 | 2 |
| | | | | | | | | | | 1.4031 | 2 |
| | | | | | | | | | | 1.3872 | 3 |
| | | | | | | | | | | 1.3557 | 2 |
| | | | | | | | | | | 1.3538 | 2 |
| | | | | | | | | | | 1.3517 | 2 |

Tabelle 5

| Beispiele | Aerosil g | $H_3BO_3$ g | eingesetztes $SiO_2/B_2O_3$ | NaOH g | gefunden $SiO_2$ % | gefunden $B_2O_3$ % | gefunden $SiO_2/B_2O_3$ | gefunden Na % |
|---|---|---|---|---|---|---|---|---|
| 7 | 80 | 7,6 | 22 | 7,35 | 90,1 | 4,65 | 22,6 | 3,08 |
| 8 | 80 | 3,6 | 44 | 3,7 | 94,9 | 0,78 | 142,5 | 1,05 |

Beispiel 9

Der in Beispiel 2 mit Diethylenglykoldimethylether hergestelte Borosilikatzeolith – ZBH wird mit Böhmit im Verhältnis 60:40 verstrangt. Der Austausch erfolgt mit $NH_4Cl$ (20%ige $NH_4Cl$-Lösung) im Verhältnis 1:15 in 2 h bei 80°C.

80%iges Methanol wird bei 450°c bei einer Belastung von WHSV=7,8 $h^{-1}$ zu 100% umgesetzt.

Die Ausbeuten, bezogen auf eingesetztes $CH_2$ sind

| | % |
|---|---|
| $Ch_4$ | 1,5 |
| $C_2H_4$ | 4,1 |
| $C_2H_6$ | 0,1 |
| $C_3H_6$ | 25,0 |
| $C_3H_8$ | 0,8 |
| $C_4$-KW | 17,6 |
| C5-KW | 46,7 |

**Patentansprüche**

1. Borosilikatzeolithe – ZBH der allgemeinen Formel

$M_{2/n}O : B_2O_3 : x\,SiO_2 : y\,H_2O,$

worin M zumindest ein Kation mit einer Wertigkeit n bedeutet, x≥10 beträgt und y zwischen 0 und 80 liegt und gemäss folgendem vereinfachtem Röntgenbeugungsdiagramm mit den wichtigsten Beugungslinien:

| Interplanarer Abstand d(Å) | Relative Intensität I/I° |
|---|---|
| 11.0746 ± 0,15 | 100 |
| 9.9725 | 68 |
| 9.7271 | 19 |
| 3.8322 | 96 |
| 3.7980 | 70 |
| 3.7282 | 30 |
| 3.6940 | 46 |
| 3.6254 | 25 |
| 1.9999 ± 0,07 | 10 |
| 1.9825 | 9 |

2. Verfahren zur Herstellung von kristallinen Borosilikatzeolithen – ZBH vom Pentasiltyp nach Anspruch 1 durch hydrothermale Kristallisation von Siliciumdioxid und Borsäure in Gegenwart von Alkalisalzen, dadurch gekennzeichnet, dass man in der Reaktionsmischung ein molares Verhältnis von $SiO_2/B_2O_3$ von 3 bis 300, bevorzugt von 10 bis 50 einstellt und die Kristallisation bei Temperaturen von 80 bis 140°C in etherischer oder wässrig-etherischer Mischung ausführt.

3. Verfahren nach Anspruch 2, dadurch gekennzeichnet, dass man als Lösungsmittel für die Kristallisation Diethylenglykoldimethylether bzw. ein wässrig-etherisches Gemisch verwendet.

4. Verfahren nach Anspruch 2, dadurch gekennzeichnet, dass man als Lösungsmittel für die Kristallisation Triethylenglykoldimethylether bzw. ein wässrig-etherisches Gemisch verwendet.

5. Verfahren nach Anspruch 2 bis 4, dadurch gekennzeichnet, dass die Mischung Alkalisalze in Mengen von 1 bis 3 Mol pro Mol $B_2O_3$ enthält.

6. Verwendung der nach Anspruch 2 bis 5 hergestellten kristallinen Borosilikatzeolithe – ZBH als Katalysator für die Herstellung von Olefinen und/oder Aromaten aus Methanol und/oder Dimethylether.

**Claims**

1. ZBH borosilicate zeolites which have the general Formula

$M_{2/n}O : B_2O_3 : x\,SiO_2 : y\,H_2O,$

where M is at least one cation having a valency n, x is≥10 and y is from 0 to 80, and in whose simplified X-ray diffraction diagram the most important diffraction lines are:

| Interplanar spacing d(A°) | Relative intensity I/I° |
|---|---|
| 11.0746 ± 0.15 | 100 |
| 9.9725 | 68 |
| 9.7271 | 19 |
| 3.8322 | 96 |
| 3.7980 | 70 |
| 3.7282 | 30 |
| 3.6940 | 46 |
| 3.6254 | 25 |
| 1.9999 ± 0.07 | 10 |
| 1.9825 | 9 |

2. A process for the preparation of crystalline ZBH borosilicate zeolites of the pentasil type as claimed in claim 1 by hydrothermal crystallization of silicon dioxide and boric acid in the presence of alkali metal salts, wherein the molar ratio of $SiO_2/B_2O_3$ employed in the reaction mixture is from 3 to 300, preferably from 10 to 50, and the crystallization is carried out at from 80 to 140°C in an ethereal or aqueous-ethereal mixture.

3. A process as claimed in claim 2, wherein the solvent used for the crystallization is diethylene glycol dimethyl ether or a mixture thereof with water.

4. A process as claimed in claim 2, wherein the solvent used for the crystallization is triethylene glycol dimethyl ether or a mixture thereof with water.

5. A process as claimed in claims 2 to 4, wherein the mixture contains from 1 to 3 moles of alkali metal salts per mole of $B_2O_3$.

6. The use of a crystalline ZBH borosilicate zeolite prepared as claimed in claims 2 to 5 as catalyst for the preparation of olefins and/or aromatics from methanol and/or dimethyl ether.

**Revendications**

1. Zéolithes de boro-silicate-ZBH de la formule générale

$M_{2/n}O : B_2O_3 : x\, SiO_2 : y\, H_2O,$

dans laquelle M désigne au moins un cation d'une valence n, x est égal ou supérieur à 10 et y vaut entre 0 et 80, dont le diagramme simplifié de diffraction des rayons X comprend les lignes de diffraction principales ci-après:

| distance interplanaire d(Å) | intensité relative I/I° |
|---|---|
| 11,0746 ± 0,15 | 100 |
| 9,9725 | 68 |
| 9,7271 | 19 |
| 3,8322 | 96 |
| 3,7980 | 70 |
| 3,7282 | 30 |
| 3,6940 | 46 |
| 3,6254 | 25 |
| 1,9999 ± 0,07 | 10 |
| 1,9825 | 9 |

2. Procédé de préparation de zéolithes de borosilicate – ZBH cristallisées du type pentasil selon la revendication 1 par cristallisation hydro-thermique de bioxyde de silicium et d'acid borique en présence de sels de métaux alcalins, caractérisé en ce que l'on règle dans le mélange réactionnel le rapport molaire $SiO_2$-$B_2O_3$ entre 3 et 300, en particulier entre 10 et 50, et on réalise la cristallisation entre 80 et 140°C dans un milieu éthéré ou éthéré aqueux.

3. Procédé suivant la revendication 2, caractérisé en ce que l'on utilise comme solvant pour la cristallisation l'éther diméthylique de diéthylèneglycol ou un mélange éther-eau.

4. Procédé suivant la revendication 2, caractérisé en ce que l'on utilise comme solvant pour la cristallisation l'éther diméthylique de triéthylèneglycol ou un mélange éther-eau.

5. Procédé suivant l'une des revendications 2 à 4, caractérisé en ce que le mélange réactionnel contient par mole de $B_2O_3$ entre 1 et 3 moles de sels de métaux alcalins.

6. Utilisation des zéolithes de boro-silicate – ZBH cristallisées, préparées (par le procédé) selon les revendications 2 à 5, comme catalyseurs pour la préparation d'oléfines et(ou) de substances aromatiques à partir d'alcool méthylique et(ou) d'éther diméthylique.